# EUROPEAN PATENT SPECIFICATION

(11) **EP 3 327 441 B1**
(45) Date of publication and mention of the grant of the patent: **17.02.2021**
(21) Application number: 16830490.5
(22) Date of filing: 25.07.2016
(51) Int. Cl.: G01N 33/68, A61K 31/519, A61K 35/14, A61K 35/28, A61K 41/00, A61P 7/00, G01N 33/53, G01N 33/543

(54) **METHOD FOR ASSISTING DIAGNOSIS OF CONDITIONS OF MYELOFIBROSIS, METHOD FOR MONITORING THERAPEUTIC EFFECT, AND A DEVICE THEREFOR**
VERFAHREN ZUR UNTERSTÜTZUNG DER DIAGNOSE VON MYELOFIBROSEERKRANKUNGEN, VERFAHREN ZUR ÜBERWACHUNG DER THERAPEUTISCHEN WIRKUNG SOWIE EINE DAFÜR ZU VERWENDENDE VORRICHTUNG
MÉTHODE D'AIDE AU DIAGNOSTIC D'AFFECTIONS DE TYPE MYÉLOFIBROSE, MÉTHODE DE SURVEILLANCE D'UN EFFET THÉRAPEUTIQUE, ET UN DISPOSITIF UTILISÉS POUR CE FAIRE

(30) Priority: 24.07.2015 JP 2015146977
(43) Date of publication of application: 30.05.2018
(73) Proprietor: Public University Corporation Nagoya City University, Mizuho-ku Nagoya-shi Aichi 467-8601 (JP); Sysmex Corporation, Kobe-shi, Hyogo 651-0073 (JP)
(72) Inventor: TANAKA, Yasuhito, Nagoya-shi Aichi 467-8601 (JP); KUSUMOTO, Shigeru, Nagoya-shi Aichi 467-8601 (JP); TAKAHAMA, Youichi, Kobe-shi Hyogo 651-0073 (JP); KAGAWA, Takashi, Kobe-shi Hyogo 651-0073 (JP)
(74) Representative: De Clercq & Partners
(86) International application number: PCT/JP2016/071746
(87) International publication number: WO 2017/018385

(56) References cited:
- WO-A1-2005/064333
- WO-A1-2011/007797
- JP-A- 2008 032 520
- JP-A- 2010 532 872
- JP-A- 2012 507 291
- BEDEKOVICS JUDIT ET AL: "Platelet derived growth factor receptor-beta (PDGFR[beta]) expression is limited to activated stromal cells in the bone marrow and shows a strong correlation with the grade of myelofibr", VIRCHOWS ARCHIV, SPRINGER INTERNATIONAL, BERLIN, DE, vol. 463, no. 1, 8 June 2013 (2013-06-08), pages 57-65, XP035373167, ISSN: 0945-6317, DOI: 10.1007/S00428-013-1434-0 [retrieved on 2013-06-08]
- ATSUSHI KUNO ET AL: "Reconstruction of a robust glycodiagnostic agent supported by multiple lectin-assisted glycan profiling", PROTEOMICS - CLINICAL APPLICATIONS, 1 August 2013 (2013-08-01), pages n/a-n/a, XP055518835, DE ISSN: 1862-8346, DOI: 10.1002/prca.201300010
- JOHN J O'SHEA ET AL: "Janus kinase inhibitors in autoimmune diseases", ANNALS OF THE RHEUMATIC DISEASES, vol. 72, no. suppl 2, 25 April 2013 (2013-04-25), pages ii111-ii115, XP055504432, GB ISSN: 0003-4967, DOI: 10.1136/annrheumdis-2012-202576
- GIANELLI U ET AL.: 'The European Consensus on grading of bone marrow fibrosis allows a better prognostication of patients with primary myelofibrosis' MOD PATHOL vol. 25, no. 9, 2012, pages 1193 - 1202, XP055349936

## Description

The present invention relates to a method for assisting the diagnosis of the condition of myelofibrosis (MF), and a method for monitoring the therapeutic effect on MF. The present invention also relates to an apparatus for executing these methods.

### BACKGROUND ART

Myelofibrosis is a type of myeloproliferative tumor and is a disease in which extensive fibrosis occurs in the bone marrow. As a result of fibrosis of the bone marrow, blood is made in sites other than the bone marrow, especially the spleen, and splenomegaly occurs. Myelofibrosis is classified into two types: primary myelofibrosis of unknown origin and secondary myelofibrosis occurring following the underlying disease. In primary myelofibrosis, stimulation of various cytokines secreted from abnormally proliferating megakaryocytes proliferate fibroblasts to produce reticular fibers and collagen fibers, and fibrosis of bone marrow progresses. Secondary myelofibrosis often migrates from hematopoietic tumors such as polycythemia vera and essential thrombocythemia, and the condition is similar to primary myelofibrosis.

In the diagnosis of myelofibrosis, fibrosis of bone marrow is confirmed by observing the bone marrow collected from the patient with a microscope (bone marrow biopsy). The condition of fibrosis is evaluated based on the semi-quantitative criteria adopted by WHO, "European consensus on grading of bone marrow fibrosis". According to the criteria, the condition of fibrosis is classified into four grades of MF-0, MF-1, MF-2 and MF-3. It is known that prognosis of a patient can be predicted according to the condition of myelofibrosis since myelofibrosis progresses parallel to the stage. For example, Non Patent Literature 1 predicts the prognosis of patients with early myelofibrosis by classifying the condition of myelofibrosis according to the above criteria.

It has been suggested that gene mutation of kinase called Janus kinase (JAK), which regulates signal transduction of cytokine, is involved in the development of myelofibrosis. In primary myelofibrosis, there are reports that mutations in the JAK2 gene are observed in about half of patients. In addition, Patent Literature 1 discloses a method of diagnosing and prognosing a myeloproliferative disease based on the measurement of the presence or absence of a predetermined mutation in a subject's JAK2 gene. O'Shea et al. (2013) have reviewed Jak inhibitors as drugs in the treatments of rheumatoid arthritis and related disorders.

Bedkovics et al. (2013) revealed that bone marrow PDGFRβ expression closely correlates with the grade of myelofibrosis.

Kuno et al. (2013) optimized a glycan-based sandwich immunoassay system for detecting the *Wisteria floribunda* agglutinin (WFA) positive human Mac-2 binding protein (WFA⁺-hM2BP) in the context of liver fibrosis. Indeed, WFA⁺-hM2BP has been confirmed as a marker glycoprotein having fibrosis-related glycol-alteration.

### CITATIONS LIST

### Patent Literature

Patent Literature 1: WO 2010/051214

### Non Patent Literature

Non Patent Literature 1: Gianelli U. et al., Modern Pathology, vol. 25, p. 1193-1202, 2012
Bedekovics et al., Virchows Arch 463 : 57-65, 2013
Kuno et al., Proteomics Clin. Appl. 7 : 642-647, 2013
O'Shea et al., Ann Rheum Dis 72 : ii111-ii115, 2013

### SUMMARY OF INVENTION

### TECHNICAL PROBLEMS

The condition of myelofibrosis is information necessary to ascertain the extent to which myelofibrosis is progressing and to confirm the effect when treatment is performed. In addition, the condition of myelofibrosis is also useful information for the prognostic prediction of patients. In the method described in Patent Literature 1 however, diagnosis of myeloproliferative disease is performed by measuring the presence or absence of a specific JAK2 mutation, but it is not possible to grasp the condition of myelofibrosis and grasp the degree of progression of myelofibrosis. Accordingly, currently only a bone marrow biopsy can be used for examining the condition of myelofibrosis. However, collection of bone marrow places a heavy burden on the patient, so it is not possible to perform bone marrow biopsies frequently to examine the condition of myelofibrosis. In addition, this evaluation of myelofibrosis requires the technique and experience of histopathological examination. Furthermore, since it is necessary to take an expensive therapeutic agent every day in medication therapy for myelofibrosis, there is a demand for examining the condition of myelofibrosis at a higher frequency in order to confirm the effectiveness of medication therapy. Therefore, it has been desired to develop a means for minimizing the burden on the patient and enabling objective evaluation of the condition of myelofibrosis.

### SOLUTIONS TO PROBLEMS

The present inventors have found that the condition of myelofibrosis can be objectively evaluated by measuring a predetermined glycoprotein contained in the peripheral blood of a myelofibrosis patient as a biomarker, thereby completing the present invention.

Therefore, the present invention provides a method for assisting the diagnosis of the condition of myelofibrosis, comprising the steps of measuring a marker protein contained in the peripheral blood collected from a patient with myelofibrosis, and determining the condition of myelofibrosis of the patient based on the measured value of this marker protein, wherein this marker protein is Mac-2-binding protein (M2BP) having a sugar chain that binds to Wisteria floribunda agglutinin (WFA) lectin.

The present invention also provides a method for monitoring the therapeutic effect on myelofibrosis, comprising the steps of measuring a marker protein contained in the peripheral blood collected from a patient with myelofibrosis who has been treated for myelofibrosis, and determining the therapeutic effect on myelofibrosis based on the measured value of this marker protein, wherein this marker protein is M2BP having a sugar chain that binds to WFA lectin.

The present invention further provides an apparatus for diagnosing the condition of myelofibrosis, comprising a computer containing a processor and a memory under the control of this processor, wherein a computer program for making the computer execute the steps of acquiring a measured value of the marker protein contained in the peripheral blood collected from a patient with myelofibrosis and determining the condition of myelofibrosis of the patient based on the measured value of this marker protein is recorded in the memory, and the marker protein is M2BP having a sugar chain that binds to WFA lectin.

The present invention further provides an apparatus for monitoring the therapeutic effect on myelofibrosis, comprising a computer containing a processor and a memory under the control of this processor, wherein a computer program for making the computer execute the steps of acquiring a measured value of the marker protein contained in the peripheral blood collected from a patient with myelofibrosis who has been treated for myelofibrosis, and determining the therapeutic effect on myelofibrosis based on the measured value of this marker protein is recorded in the memory, and the marker protein is M2BP having a sugar chain that binds to WFA lectin.

### ADVANTAGEOUS EFFECTS OF INVENTION

According to the present invention, it is possible to minimize the burden on a patient in collecting a sample, and objectively evaluate the condition of myelofibrosis.

### BRIEF DESCRIPTION OF DRAWINGS

Fig. 1 are box plots showing measured values of WFA⁺-M2BP in each of the MF-0/1 group and the MF-2/3 group classified by bone marrow biopsy.
Fig. 2 is an ROC curve created for calculating a cut-off value of measured values of WFA⁺-M2BP.
Fig. 3 are box plots showing measured values of WFA⁺-M2BP in each of MF-0 group, MF-1 group, MF-2 group and MF-3 group classified by bone marrow biopsy.
Fig. 4 is a graph showing changes in measured value of WFA⁺-M2BP in myelofibrosis patients by administration of a JAK inhibitor.
Fig. 5 is a schematic diagram showing an example of an apparatus for diagnosing the condition of myelofibrosis.
Fig. 6 is a block diagram showing a hardware configuration of an apparatus for diagnosing the condition of myelofibrosis.
Fig. 7 is a flowchart for determination of the condition of myelofibrosis using an apparatus for diagnosing the condition of myelofibrosis.
Fig. 8 is a flowchart for prognosis determination using an apparatus for predicting the prognosis of myelofibrosis.
Fig. 9 is a flowchart for determination of a therapeutic effect using an apparatus for monitoring the therapeutic effect on myelofibrosis.
Fig. 10A is a graph showing a change in measured values of WFA⁺-M2BP in myelofibrosis patient A by administration of a JAK inhibitor.
Fig. 10B is a graph showing a change in measured values of WFA⁺-M2BP in myelofibrosis patient B by administration of a JAK inhibitor.
Fig. 10C is a graph showing a change in measured values of WFA⁺-M2BP in myelofibrosis patient C by administration of a JAK inhibitor.
Fig. 11A are micrographs when bone marrow collected from myelofibrosis patient A before and after administration of a JAK inhibitor was stained with hematoxylin (HE), silver, or Masson's trichrome.
Fig. 11B are micrographs of bone marrow collected from myelofibrosis patient C before and after administration of a JAK inhibitor, stained with HE, silver, or Masson's trichrome.

### DESCRIPTION OF EMBODIMENTS

### [1. Method for Diagnosing Condition of Myelofibrosis]

In the method for diagnosing the condition of myelofibrosis of the present invention (hereinafter also simply referred to as "diagnostic method"), first, as a marker protein contained in the peripheral blood collected from a patient with myelofibrosis, M2BP having a sugar chain that binds to WFA lectin is measured.

A patient with myelofibrosis (hereinafter also referred to as "MF patient") may be a person diagnosed as primary myelofibrosis or secondary myelofibrosis based on known diagnostic criteria. Regarding the MF patient, age, sex, symptom, basic disease, treatment history, mutation of genes involved in myelofibrosis (such as JAK2 gene) and the like are not particularly limited.

As a blood sample, peripheral blood collected from a MF patient may be used, or plasma or serum prepared from the peripheral blood may be used. Serum is preferred among them. In embodiments, the blood sample may be diluted with an appropriate aqueous medium, as necessary. Such an aqueous medium is not particularly limited as long as it does not interfere with the measurement described later, and examples thereof include water, physiological saline, a buffer solution, and the like. The buffer solution is not particularly limited as long as it has a buffering effect at a pH near neutrality (for example, a pH of 6 or more and 8 or less). Examples of the buffer solution include Good buffers such as HEPES, MES, Tris and PIPES, phosphate buffered saline (PBS), and the like.

The marker protein measured in the present method is M2BP having a sugar chain that binds to WFA lectin (hereinafter also referred to as "WFA⁺-M2BP"). A protein having a sugar chain like M2BP is called a glycoprotein. It is generally known that glycoproteins differ in the structure of sugar chains depending on the type or condition of tissues and cells to be produced. The present inventors have found that WFA⁺-M2BP hardly exists in blood samples obtained from healthy subjects, but WFA⁺-M2BP exists in blood samples obtained from MF patients, and the measured value of WFA⁺-M2BP in the sample is correlated with the condition of myelofibrosis of the MF patients, particularly, patients in the MF-0/1 group with good prognosis (low risk) and patients in the MF-2/3 group with poor prognosis (high risk) can be classified by a comparison of the measured value of WFA⁺-M2BP and the cut-off value.

A means for measuring WFA⁺-M2BP is not particularly limited as long as it can acquire a value reflecting the amount or concentration of WFA⁺-M2BP (hereinafter also referred to as "measured value of WFA⁺-M2BP") contained in a blood sample. In the present method, a method of capturing WFA⁺-M2BP by using a substance capable of specifically binding to a sugar chain of WFA⁺-M2BP (that is, a sugar chain that binds to WFA lectin) is preferable. The WFA⁺-M2BP contained in the blood sample may be measured by detecting WFA⁺-M2BP captured by such a substance by a method known in the art. WFA lectin is particularly preferable as the substance capable of specifically binding to a sugar chain of WFA⁺-M2BP. WFA lectin itself is known as lectin contained in seeds of Wisteria and is generally available. In addition, as with WFA lectin, it is also possible to use an antibody capable of specifically binding to a sugar chain of WFA⁺-M2BP.

Naturally occurring WFA lectin is a tetrameric protein composed of four subunits. From this tetrameric WFA lectin, a monomer or dimeric WFA lectin can be obtained by a predetermined treatment using a reducing agent or the like. In the present specification, unless otherwise specified, the notations "WFA" and "WFA lectin" intend both monomeric WFA lectin and multimeric WFA lectin. In addition, in the present specification, when referring to a WFA lectin composed of a predetermined number of subunits, for example, as in "monomeric WFA", "dimeric WFA" and "tetrameric WFA", the number of subunits is clearly written.

In embodiments, the WFA lectin may be a tetrameric WFA or may be a monomeric WFA or a dimeric WFA obtained from a tetrameric WFA. Among them, a dimeric WFA is preferable from the point of high reactivity with WFA⁺-M2BP.

From the viewpoint of specifying and measuring M2BP from substances specifically binding to WFA lectin, WFA⁺-M2BP contained in a blood sample is preferably measured using an anti-M2BP antibody, in addition to WFA lectin. Specifically, first, a blood sample, WFA lectin and an anti-M2BP antibody are mixed to form a complex containing WFA⁺-M2BP, WFA lectin and an anti-M2BP antibody. In this complex, the WFA lectin binds to the sugar chain of WFA⁺-M2BP, and the anti-M2BP antibody binds to the protein portion of WFA⁺-M2BP.

The anti-M2BP antibody is not particularly limited as long as it can specifically bind to the protein portion of WFA⁺-M2BP. The anti-M2BP antibody may be any of monoclonal antibodies, polyclonal antibodies, and fragments thereof (for example, Fab, F(ab')2, etc.). Alternatively, a commercially available anti-M2BP antibody may be used.

The order of mixing the blood sample, the WFA lectin and the anti-M2BP antibody is not particularly limited, and these may be mixed substantially simultaneously or sequentially mixed.

In the present method, it is preferable to form a complex containing WFA⁺-M2BP, WFA lectin and anti-M2BP antibody on a solid phase. For example, a solution containing the above complex may be brought into contact with a solid phase capable of capturing the WFA lectin or the anti-M2BP antibody. Alternatively, the WFA lectin or the anti-M2BP antibody may be previously immobilized on a solid phase. For example, a solid phase on which the WFA lectin (or anti-M2BP antibody) is immobilized, a blood sample, and an anti-M2BP antibody (or WFA lectin) are contacted with each other, whereby the complex can be formed on the solid phase. Then, the complex formed on the solid phase is detected by a method known in the art, whereby the amount or concentration of WFA⁺-M2BP contained in the blood sample can be measured.

The mode of immobilization of WFA lectin or anti-M2BP antibody on the solid phase is not particularly limited. For example, the WFA lectin or the anti-M2BP antibody may be directly bound to the solid phase, or the WFA lectin or anti-M2BP antibody and the solid phase may be indirectly bound via another substance. Examples of a direct bond include physical adsorption and the like. Examples of an indirect bond include a bond via a combination of biotin and avidin or streptavidin (hereinafter also referred to as "avidin"). In this case, by previously modifying WFA lectin or anti-M2BP antibody with biotin and previously binding the avidin to the solid phase, the WFA lectin or the anti-M2BP antibody and the solid phase can be indirectly bound through the binding between the biotin and the avidin.

The material of the solid phase is not particularly limited, and it can be selected from, for example, organic polymer compounds, inorganic compounds, biopolymers, and the like. Examples of the organic polymer compound include latex, polystyrene, polypropylene, and the like. Examples of the inorganic compound include magnetic bodies (iron oxide, chromium oxide, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, membranes, microplates, microtubes, test tubes, and the like. Among them, particles are preferable, and magnetic particles are particularly preferable.

In the embodiments, B/F separation for removing an unreacted free component not forming a complex may be performed between the formation of the complex and the measurement described later. The unreacted free component refers to a component not constituting a complex. Examples thereof include WFA lectin or anti-M2BP antibodies not bound to WFA⁺-M2BP, and the like. The means of B/F separation is not particularly limited, and when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. When the solid phase is a magnetic particle, B/F separation can be performed by aspirating and removing a liquid containing an unreacted free component by a nozzle while magnetically constraining the magnetic particles with a magnet, which is preferable from the viewpoint of automation. After removing the unreacted free component, the solid phase capturing the complex may be washed with a suitable aqueous medium such as PBS.

In the present method, it is preferable to measure WFA⁺-M2BP contained in a blood sample using the WFA lectin immobilized on magnetic particles and an anti-M2BP antibody labeled with a labeling substance (hereinafter also referred to as "labeled anti-M2BP antibody"). By using the magnetic particles on which WFA lectin is immobilized, the reactivity of WFA lectin with WFA⁺-M2BP in the sample is improved.

The WFA lectin to be immobilized on magnetic particles is preferably a dimeric WFA obtained by dimerizing a tetrameric WFA. The dimeric WFA can be obtained, for example, by dissociating subunits of a tetrameric WFA using a sulfhydryl reagent or a reducing agent. Also, by bringing a crosslinking agent into contact with a tetrameric WFA, the tetrameric WFA can be converted to a dimeric WFA. As such a crosslinking agent, a crosslinking agent that forms a crosslink with the amino group in the tetrameric WFA is preferable. Examples of a reactive group for the amino group include crosslinking agents having at least one functional group selected from the group consisting of an N-hydroxysuccinimide ester group, an isothiocyano group, a chlorosulfone group, a chlorocarbonyl group, an oxyethylene group, a chloroalkyl group having 1 to 4 carbon atoms, an aldehyde group and a carboxyl group. By using such a crosslinking agent, the tetrameric WFA can be efficiently converted to a dimeric WFA.

The molar ratio (WFA/crosslinking agent) when mixing the tetrameric WFA and the crosslinking agent is preferably 1/10 or less, and more preferably 1/20 or less. On the other hand, the lower limit of the molar ratio (WFA/crosslinking agent) can be set to 1/100 or more, in consideration of the balance between the amount of the crosslinking agent used and the yield of the dimeric WFA to be produced.

In the case where WFA lectin is immobilized on magnetic particles through the binding between biotin and avidin, biotinylated dimeric WFA may be used. The biotinylated dimeric WFA can be obtained, for example, by dimerizing tetrameric WFA lectin using a crosslinking agent containing biotin. The crosslinking agent containing biotin can be obtained, for example, by binding biotin and the reactive group of the crosslinking agent through a spacer. Such a spacer is not particularly limited, but examples thereof include compounds having an aminohexanoyl group (aminocaproyl group) and the like.

The labeling substance used for the labeled anti-M2BP antibody is not particularly limited as long as a detectable signal is generated. For example, it may be a substance which itself generates a signal (hereinafter also referred to as "signal generating substance") or a substance which catalyzes the reaction of other substances to generate a signal. Examples of the signal generating substance include fluorescent substances, radioactive isotopes, and the like. Examples of the substance that catalyzes the reaction of other substances to generate a detectable signal include enzymes. Examples of the enzymes include alkaline phosphatase, peroxidase, β-galactosidase, luciferase, and the like. Examples of the fluorescent substances include fluorescent dyes such as fluorescein isothiocyanate (FITC), rhodamine and Alexa Fluor (registered trademark), fluorescent proteins such as GFP, and the like. Examples of the radioisotopes include ¹²⁵I, ¹⁴C, ³²P, and the like. Among them, an enzyme is preferable as a labeling substance, and alkaline phosphatase is particularly preferable.

The labeled anti-M2BP antibody is obtained by labeling an anti-M2BP antibody with the above labeling substance by a labeling method known in the art. Labeling may also be performed using a commercially available labeling kit or the like.

By detecting a signal generated by the labeling substance of the labeled anti-M2BP antibody contained in the complex, a measured value reflecting the amount or concentration of WFA⁺-M2BP contained in the blood sample can be acquired. The phrase "detecting a signal" herein includes qualitatively detecting the presence or absence of a signal, quantifying a signal intensity, and semi-quantitatively detecting the intensity of a signal. Semi-quantitative detection means to show the intensity of the signal in stages such as "no signal generated", "weak", "medium", "strong", and the like. In the present embodiment, it is preferable to detect the intensity of a signal quantitatively or semi-quantitatively.

Methods for detecting a signal themselves are known in the art. A measurement method according to the type of signal derived from the labeling substance may be appropriately selected. For example, when the labeling substance is an enzyme, signals such as light and color generated by reacting a substrate for the enzyme can be measured by using a known apparatus such as a spectrophotometer.

The substrate of the enzyme can be appropriately selected from known substrates according to the type of the enzyme. For example, when alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl )phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate.

When the labeling substance is a radioactive isotope, radiation as a signal can be measured using a known apparatus such as a scintillation counter. Also, when the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The detection result of the signal can be used as the measured value of WFA⁺-M2BP. For example, when quantitatively detecting the intensity of a signal, the measured value itself of the signal intensity or the value calculated from the measured value can be used as the measured value of WFA⁺-M2BP. Examples of the value calculated from the measured value of the signal intensity include a value obtained by subtracting the measured value of the negative control sample from the measured value, a value obtained by dividing the measured value by the measured value of the positive control sample, combinations thereof, and the like. Examples of the negative control sample include blood samples that do not contain WFA⁺-M2BP, such as healthy subject serum. Examples of the positive control sample include blood samples containing WFA⁺-M2BP at a predetermined concentration.

Next, in the diagnostic method of the present invention, the condition of myelofibrosis of a MF patient is determined based on the above measured value of WFA⁺-M2BP.

The above determination is preferably performed by deciding to which stage, among a plurality of stages set in accordance with the degree of progression of myelofibrosis, the condition of myelofibrosis of the MF patient corresponds. As such a stage, a grade or the like defined in the determination criteria of myelofibrosis known in the art may be used. Preferred examples include the grades (MF-0, MF-1, MF-2 and MF-3) defined in "European consensus on grading of bone marrow fibrosis". Each grade of MF-0 to MF-3 is based on histopathological findings of bone marrow collected from the MF patients. MF-0 corresponds to normal bone marrow and refers to the bone marrow in which no intersection of reticular fibers is recognized. MF-1 refers to the bone marrow around which intersections and a loose network of reticular fibers are found around blood vessels. MF-2 refers to the bone marrow in which intersections of reticular fibers are diffusely recognized at high density, and bundles of collagen fibers and findings of osteosclerosis are locally found. MF-3 refers to the bone marrow in which intersections of reticular fibers are diffusely recognized at high density, and thick and coarse bundles of collagen fibers and distinctive findings of osteosclerosis are found.

The number of the above stages is not particularly limited, but usually it may be 2, 3 or 4 stages. For example, when the condition of myelofibrosis is determined in 2 stages, the condition of myelofibrosis may be determined whether it is a grade corresponding to MF-0/1 or a grade corresponding to MF-2/3, among the grades of myelofibrosis represented by MF-0, MF-1, MF-2 and MF-3. Here, "MF-0/1" is intended to be either MF-0 or MF-1, and corresponds to the pre-fibrosis stage. "MF-2/3" is intended to be either MF-2 or MF-3, and corresponds to the fibrosis stage. In addition, in the case where the condition of myelofibrosis is determined in 4 stages, to which grade the condition of myelofibrosis corresponds can be determined, among the grades of myelofibrosis represented by MF-0, MF-1, MF-2 and MF-3.

In the present method it is preferable to compare the measured value of WFA⁺-M2BP with a predetermined cut-off value and determine the condition of myelofibrosis based on the comparison result. For example, in the case where the condition of myelofibrosis is determined in 2 stages, when the measured value of WFA⁺-M2BP is equal to or higher than a predetermined cut-off value, the condition of myelofibrosis is determined to be a grade corresponding to MF-2/3. Conversely, when the measured value of WFA⁺-M2BP is lower than the predetermined cut-off value, the condition of myelofibrosis is determined to be a grade corresponding to MF-0/1.

The above predetermined cut-off value is not particularly limited, and can be set empirically for MF patients, for example, by accumulating data on bone marrow biopsy and the measured values of WFA⁺-M2BP in the peripheral blood. Alternatively, the predetermined cut-off value may be set as follows. Peripheral blood is collected from MF patients of each grade of MF-0, MF-1, MF-2 and MF-3 classified by bone marrow biopsy, and the measured value of WFA⁺-M2BP is acquired.

Then, a value that can distinguish between MF-0, MF-1, MF-2 and MF-3 or a value that can distinguish between MF-0/1 and MF-2/3 is obtained from the acquired value, and the value is set as a predetermined cut-off value.

### [2. Method for Assisting Prognostic Prediction Diagnosis of Myelofibrosis]

As described above, in myelofibrosis, prognosis of a patient can be predicted according to the condition of myelofibrosis since myelofibrosis progresses parallel to the stage. Therefore, when the condition of myelofibrosis of a MF patient can be determined by the method for diagnosing the condition of myelofibrosis of the present invention, it is also possible to predict the prognosis of the patient. Accordingly, also disclosed herein is a method for assisting the prediction of prognosis of myelofibrosis (hereinafter also simply referred to as "prediction method").

In the prediction methoddisclosed herein, first, WFA⁺-M2BP is measured as a marker protein contained in the peripheral blood collected from the MF patient. The MF patient and measurement of WFA⁺-M2BP in the prediction method disclosed herein are the same as described in the diagnostic method of the present invention.

Next, in the prediction method disclosed herein, the prognosis of the MF patient is predicted based on the measured value of WFA⁺-M2BP. Here, the prediction of prognosis of an MF patient may be interpreted as prediction of the level of risk that affects the survival period of the patient. That is, when an MF patient is predicted to be at high risk, the prognosis of the MF patient is poor, and the survival period is expected to be relatively short. Also, when an MF patient is predicted to be at low risk, the prognosis of the MF patient is good, and the survival period is expected to be relatively long.

It is preferable to compare the measured value of WFA⁺-M2BP with a predetermined cut-off value and predict the prognosis of the MF patient based on the comparison result. For example, when the measured value of WFA⁺-M2BP is equal to or higher than the predetermined cut-off value, the MF patient is predicted to be at high risk. Conversely, when the measured value of WFA⁺-M2BP is lower than the predetermined cut-off value, the MF patient is predicted to be at low risk. The predetermined cut-off value can be set in the same manner as described in the diagnostic method of the present invention.

### [3. Method for Monitoring Therapeutic Effect on Myelofibrosis]

In the art, cure of myelofibrosis and prolongation of survival period are included in the therapeutic goal of myelofibrosis. Cure of myelofibrosis refers to a state in which abnormal cells and fibrosis in the bone marrow disappear. In the diagnostic method of the present invention, since the condition of myelofibrosis can be determined based on the measured value of WFA⁺-M2BP, it is possible to monitor whether or not fibrosis of bone marrow is improved by the treatment, by comparing the measured values of WFA⁺-M2BP before and after the treatment. Accordingly, the the present invention also includes a method for monitoring the therapeutic effect on myelofibrosis (hereinafter also simply referred to as "monitoring method").

In the monitoring method of the present invention, first, WFA⁺-M2BP is measured as a marker protein contained in the peripheral blood collected from a patient with myelofibrosis who has been treated for myelofibrosis.

The MF patient is not particularly limited as long as they are treated for myelofibrosis. Treatment for myelofibrosis is not particularly limited, and may be a treatment known in the art, or a treatment whose efficacy on myelofibrosis is unknown. Treatment for myelofibrosis may be either drug therapy or non-drug therapy. Examples of drug therapy known in the art include treatment using a therapeutic agent for myelofibrosis known in the art such as hydroxyurea, alkylating agents (for example, melphalan and the like), immunomodulators (for example, thalidomide, lenalidomide and the like) and JAK inhibitors (for example, ruxolitinib and the like). Among them, treatment using a JAK inhibitor is preferable, and treatment using ruxolitinib is particularly preferable. Examples of non-drug therapy known in the art include blood transfusion therapy, bone marrow transplantation, splenectomy, radiation therapy, and the like.

It is preferable to continuously collect the peripheral blood of an MF patient and measure WFA⁺-M2BP, in order to monitor the therapeutic effect. The timing and frequency of blood collection and the like are not particularly limited and can be properly determined, but it is preferable to carry it out at least every 3 months, and it may be carried out at intervals shorter than 3 months as necessary. Generally, since MF patients need to visit a hospital for follow-up for a while after the start of treatment, peripheral blood may be collected at the time of this visit. Also, when comparing the conditions of myelofibrosis before and after treatment, the peripheral blood may be collected before treatment.

Measurement of WFA⁺-M2BP in the monitoring method of the present invention is the same as described in the diagnostic method of the present invention. Measurement of WFA⁺-M2BP may be performed each time blood is collected. Alternatively, the collected blood or the plasma or serum prepared therefrom may be stored, and measurement for the stored blood sample may be performed at any time.

Next, in the monitoring method of the present invention, the therapeutic effect on myelofibrosis is determined based on the measured value of WFA⁺-M2BP. In the present embodiment, the therapeutic effect may be determined using the measured value of WFA⁺-M2BP or may be determined using the condition of myelofibrosis determined based on the measured value of WFA⁺-M2BP.

When the therapeutic effect is determined using the measured value of WFA⁺-M2BP, it is preferable to perform the determination by comparing the measured value of WFA⁺-M2BP (preferably, the current measured value) with the measured value of WFA⁺-M2BP measured in the past. As the measured value of WFA⁺-M2BP measured in the past, it is possible to use the measured value of WFA⁺-M2BP obtained by previously measuring the blood sample acquired from an MF patient in the past. Alternatively, the blood sample acquired from an MF patient in the past is stored, and the stored blood sample is measured together with the current blood sample of the MF patient, thereby the measured value acquired simultaneously with the measured value of current WFA⁺-M2BP may be used.

For example, it can be determined that a therapeutic effect is recognized when the measured value of current WFA⁺-M2BP is decreased from the measured value of past WFA⁺-M2BP. In addition, when the condition determined from the measured value of past WFA⁺-M2BP is the condition corresponding to MF-2/3 and the condition determined from the measured value of current WFA⁺-M2BP is the condition corresponding to MF-0/1, it can be determined that a remarkable therapeutic effect is recognized.

When the therapeutic effect is determined using the condition of myelofibrosis, for example, it may be performed by determining whether the condition of myelofibrosis corresponds to the pre-fibrosis stage or the fibrosis stage. Alternatively, the therapeutic effect may be determined by comparing the condition of myelofibrosis determined based on the measured value of WFA⁺-M2BP with the result of bone marrow biopsy performed at the time of diagnosis of myelofibrosis.

### [4. Marker]

Further disclosed herein is a marker for determining the condition of myelofibrosis. (Hereinafter also simply referred to as "marker"). Such a marker is present in the peripheral blood of a patient with myelofibrosis and comprises M2BP having a sugar chain that binds to WFA lectin. The marker in the blood sample derived from a MF patient can be measured, and the condition of myelofibrosis of the patient can be determined based on the obtained measured value. Measurement of the marker (i.e., WFA⁺-M2BP) and determination of the condition of myelofibrosis are the same as those described above. Furthermore, the use of M2BP having a sugar chain that binds to WFA lectin for determining the condition of myelofibrosis is also disclosed herein.

### [5. Apparatus]

Further disclosed herein are apparatuses for carrying out the methods described herein. Such apparatuses are an apparatus for diagnosing the condition of myelofibrosis (hereinafter also simply referred to as "diagnostic apparatus"), an apparatus for predicting the prognosis of myelofibrosis (hereinafter also simply referred to as "prediction apparatus"), and an apparatus for monitoring the therapeutic effect on myelofibrosis (hereinafter also simply referred to as "monitoring apparatus").

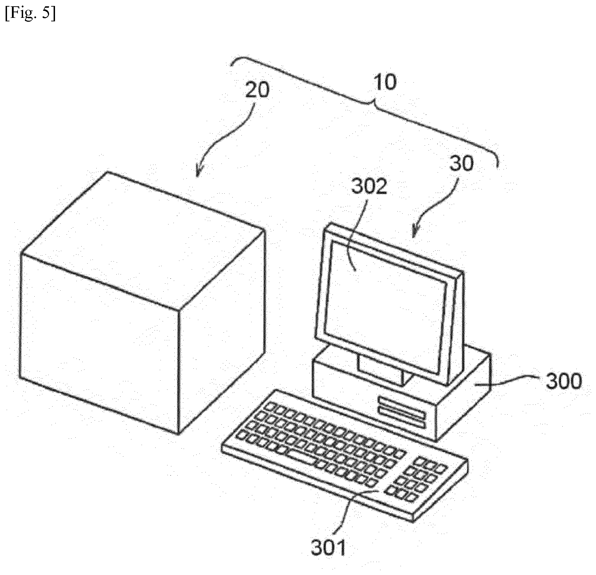

Hereinbelow, an example of the apparatus for carrying out the method as described herein will be described with reference to the drawings. However, the present invention is not limited only to the embodiment shown in this example. Fig. 5 is a schematic diagram of an apparatus for diagnosing the condition of myelofibrosis of a MF patient. A diagnostic apparatus 10 shown in Fig. 5 includes an immunoassay device 20 and a computer system 30 connected to the immunoassay device 20. The hardware configurations of the prediction apparatus and the monitoring apparatus are also the same as that of the diagnostic apparatus.

The type of immunoassay device is not particularly limited, and it can be appropriately selected according to the method of measuring WFA⁺-M2BP. In the example shown in Fig. 5, the immunoassay device 20 is a commercially available automated immunoassay device capable of detecting a chemiluminescent signal generated by a sandwich ELISA method using magnetic particles on which WFA lectin is immobilized and an enzyme-labeled anti-M2BP antibody. The immunoassay device 20 is not particularly limited as long as it can detect a signal based on the used labeling substance, and it can be appropriately selected according to the type of the labeling substance. The immunoassay device 20 may comprise a device for mixing a blood sample, a reagent containing a solid phase on which WFA lectin is immobilized and a reagent containing a labeled anti-M2BP antibody to perform an antigen-antibody reaction.

When a reagent containing magnetic particles on which WFA lectin is immobilized, a reagent containing an enzyme-labeled anti-M2BP antibody and a serum collected from a patient are set in the immunoassay device 20, the immunoassay device 20 performs an antigen-antibody reaction using each reagent, acquires a chemiluminescent signal as optical information based on the enzyme-labeled antibody specifically bound to WFA⁺-M2BP, and transmits the obtained optical information to the computer system 30.

The computer system 30 includes a computer main body 300, an input unit 301, and a display unit 302 that displays specimen information, a determination result, and the like. The computer system 30 receives the optical information from the immunoassay device 20. Then, a processor of the computer system 30 executes a program for determining the condition of fibrosis in a MF patient, installed in a hard disk 313, based on the optical information. As shown in Fig. 5, the computer system 30 may be equipment separate from the immunoassay device 20, or may be equipment including the immunoassay device 20. In the latter case, the computer system 30 may itself be the diagnostic apparatus 10. Except that, in the prediction apparatus, a program for prognostic prediction diagnosis in a MF patient is installed on the hard disk 313, and in the monitoring apparatus, a program for determining the therapeutic effect in a MF patient is installed on the hard disk 313, both have the same configuration as the diagnostic apparatus 10. In addition, the above fibrosis condition determination program, prognostic prediction program and/or therapeutic effect determination program may be installed in a commercially available automated immunoassay device.

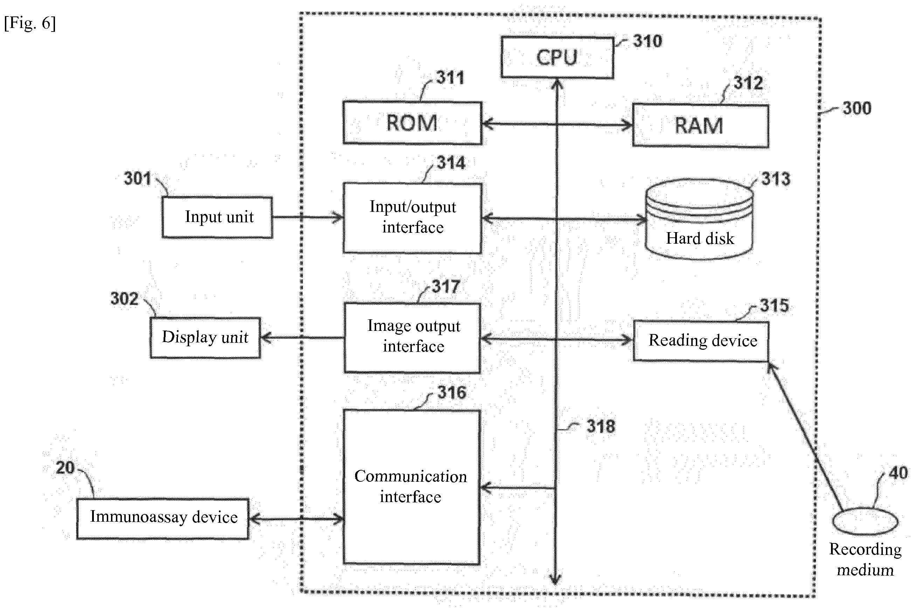

With reference to Fig. 6, the computer main body 300 includes a CPU (Central Processing Unit) 310, a ROM (Read Only Memory) 311, a RAM (Random Access Memory) 312, a hard disk 313, an input/output interface 314, a reading device 315, a communication interface 316, and an image output interface 317. The CPU 310, the ROM 311, the RAM 312, the hard disk 313, the input/output interface 314, the reading device 315, the communication interface 316 and the image output interface 317 are data-communicably connected by a bus 318. Further, the immunoassay device 20 is communicably connected to the computer system 30 via the communication interface 316.

The CPU 310 can execute a program stored in the ROM 311 or the hard disk 313 and a program loaded in the RAM 312. The CPU 310 calculates the measured value of WFA⁺-M2BP, reads the predetermined cut-off value stored in the ROM 311 or the hard disk 313, and determines the condition of myelofibrosis of the MF patient. The CPU 310 outputs the determination result and displays it on the display unit 302.

The ROM 311 includes a mask ROM, PROM, EPROM, EEPROM, and the like. In the ROM 311, a computer program executed by the CPU 310 and data used for executing the computer program are recorded as described above. In the ROM 311, data used for a determination flow to be described later such as the predetermined cut-off value, the measured value of past WFA⁺-M2BP may be recorded.

The RAM 312 includes SRAM, DRAM, and the like. The RAM 312 is used for reading the program recorded in the ROM 311 and the hard disk 313. The RAM 312 is also used as a work area of the CPU 310 when executing these programs.

In the hard disk 313, computer programs such as an operating system and an application program (the above-described fibrosis condition determination program, prognostic prediction diagnosis program and/or therapeutic effect determination program) for making the CPU 310 execute, and data used for executing the computer program are installed. In the hard disk 313, data used for a determination flow to be described later such as the predetermined cut-off value and the measured value of past WFA⁺-M2BP may be recorded.

The reading device 315 is constituted of a flexible disk drive, a CD-ROM drive, a DVD-ROM drive, and the like. The reading device 315 can read a program or data recorded in a portable recording medium 40.

The input/output interface 314 includes, for example, a serial interface such as USB, IEEE1394 or RS-232C, a parallel interface such as SCSI, IDE or IEEE1284, and an analog interface including a D/A converter, an A/D converter and the like. The input unit 301 such as a keyboard and a mouse is connected to the input/output interface 314. An operator can input various commands to the computer main body 300 through the input unit 301.

The communication interface 316 is, for example, an Ethernet (registered trademark) interface or the like. The computer main body 300 can also transmit print data to a printer or the like through the communication interface 316.

The image output interface 317 is connected to the display unit 302 including an LCD, a CRT, and the like. As a result, the display unit 302 can output a video signal corresponding to the image data coming from the CPU 310. The display unit 302 displays an image (screen) according to the input video signal.

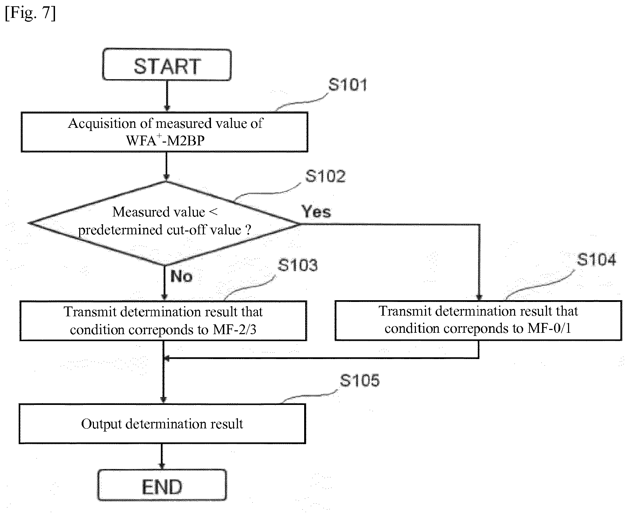

With reference to Fig. 7, a determination flow of the condition of myelofibrosis in a MF patient executed by the diagnostic apparatus 10 will be described. Here, a case where a measured value of WFA⁺-M2BP is acquired from a chemiluminescent signal generated by a sandwich ELISA method using magnetic particles on which WFA lectin is immobilized and an enzyme-labeled anti M2BP antibody and the determination is made using the acquired measured value will be described as an example. However, the present invention is not limited only to this example.

In step S101, the CPU 310 acquires optical information (chemiluminescent signal) from the immunoassay device 20, calculates the measured value of WFA⁺-M2BP from the acquired optical information, and stores it in the hard disk 313. In step S102, the CPU 310 compares the calculated WFA⁺-M2BP measured value with the predetermined cut-off value stored in the hard disk 313. When the measured value of WFA⁺-M2BP is equal to or higher than the predetermined cut-off value, the process proceeds to step S103, and the determination result indicating that the condition of myelofibrosis corresponds to MF-2/3 is stored in the hard disk 313. On the other hand, when the measured value of WFA⁺-M2BP is lower than the predetermined cut-off value, the process proceeds to step S104, and the determination result indicating that the condition of myelofibrosis corresponds to MF-0/1 is stored in the hard disk 313. In step S105, the CPU 310 outputs the determination result, and displays it on the display unit 302, or makes a printer to print the determination result. Accordingly, it is possible to provide doctors and the like with information to assist the diagnosis of the condition of myelofibrosis of MF patients.

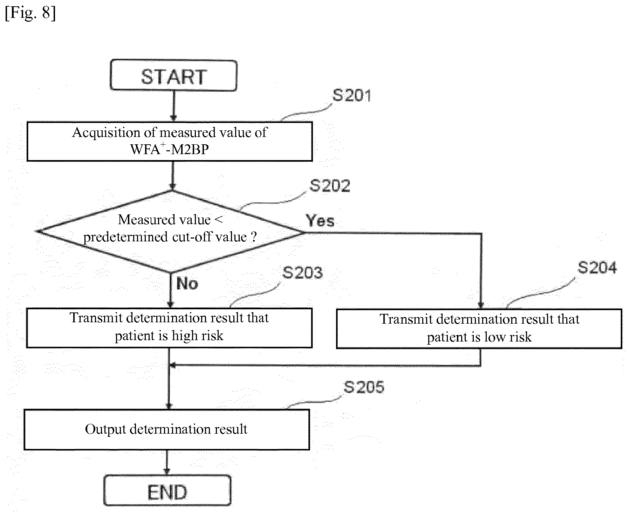

With reference to Fig. 8, a prognostic prediction flow of an MF patient executed by the prediction apparatus as disclosed herein will be described. Step S201 is the same as step S101, and the measured value of WFA⁺-M2BP is acquired from the chemiluminescent signal generated by the above sandwich ELISA method. In step S202, the CPU 310 compares the measured value of WFA⁺-M2BP calculated in step S201 with the predetermined cut-off value stored in the hard disk 313. When the measured value of WFA⁺-M2BP is equal to or higher than the predetermined cut-off value, the process proceeds to step S203, and the determination result indicating that the MF patient is high risk (poor prognosis) is stored in the hard disk 313. On the other hand, when the measured value of WFA⁺-M2BP is lower than the predetermined cut-off value, the process proceeds to step S204, and the determination result indicating that the MF patient is low risk (good prognosis) is stored in the hard disk 313. In step S205, the CPU 310 outputs the determination result, and displays it on the display unit 302, or makes a printer to print the determination result. Accordingly, it is possible to provide doctors and the like with information to assist the prognostic prediction of MF patients.

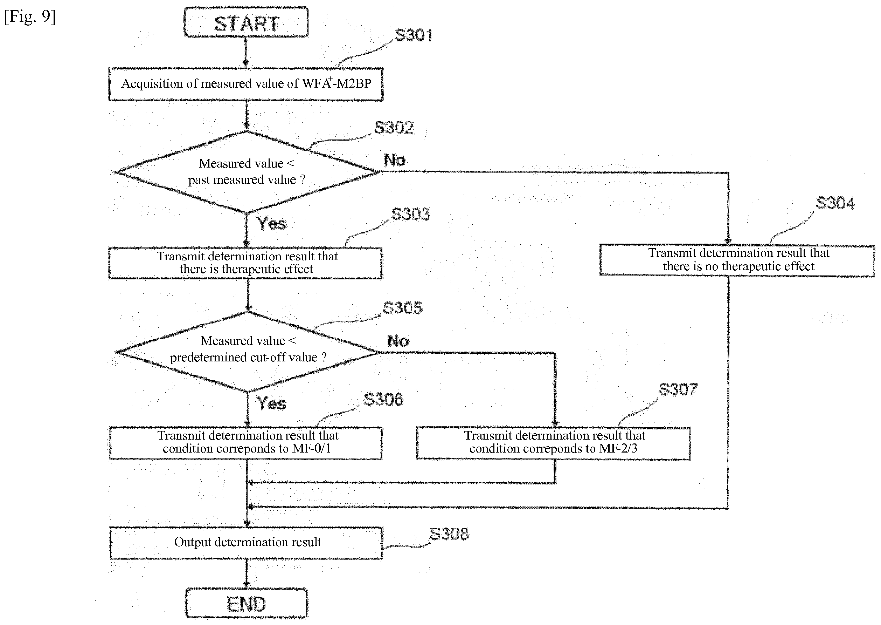

With reference to Fig. 9, a determination flow of the therapeutic effect on myelofibrosis executed by the monitoring apparatus of the present invention will be described. In this example, as the measured value of WFA⁺-M2BP measured in the past (hereinafter also referred to as "past measured value"), it will be explained about the case of determining using a measured value obtained by previously measuring a blood sample acquired in the past. However, the present invention is not limited only to this example. As a past measured value, a blood sample acquired from an MF patient in the past was preserved, and the preserved blood sample was measured together with the current blood sample of the MF patient, whereby the measured value acquired simultaneously with the current measured value may be used.

Step S301 is the same as step S101, and the measured value of WFA⁺-M2BP is acquired from the chemiluminescent signal generated by the above sandwich ELISA method. In step S302, the CPU 310 compares the measured value of WFA⁺-M2BP calculated in step S301 with the past measured value stored in the hard disk 313. When the measured value of WFA⁺-M2BP is lower than the past measured value, the process proceeds to step S303, and the determination result indicating that the treatment received by the MF patient is effective is stored in the hard disk 313. On the other hand, when the measured value of WFA⁺-M2BP is equal to or higher than the past measured value, the process proceeds to step S304, and the determination result indicating that the treatment received by the MF patient is ineffective is stored in the hard disk 313. In step S308, the CPU 310 may output the above determination result and terminate the flow.

When it is determined that there is a therapeutic effect, the condition of myelofibrosis may be further determined. With reference to Fig. 9, in step S305, the CPU 310 compares the calculated WFA⁺-M2BP measured value with the predetermined cut-off value stored in the hard disk 313. When the measured value of WFA⁺-M2BP is lower than the predetermined cut-off value, the process proceeds to step S306, and the determination result indicating that the condition of myelofibrosis corresponds to MF-0/1 is stored in the hard disk 313. On the other hand, when the measured value of WFA⁺-M2BP is equal to or higher than the predetermined cut-off value, the process proceeds to step S307, where the determination result indicating that the condition of myelofibrosis corresponds to MF-2/3 is stored in the hard disk 313. In step S308, the CPU 310 outputs the determination result, and displays it on the display unit 302, or makes a printer to print the determination result. Accordingly, it is possible to provide doctors and the like with information to assist the determination of the effect of treatment on myelofibrosis.

Further disclosed herein is the use of the above immunoassay device and computer for manufacturing an apparatus for diagnosing the condition of myelofibrosis. Thus, disclosed herein is:
the use of an immunoassay device and a computer for manufacturing an apparatus for diagnosing the condition of myelofibrosis,
the immunoassay device comprising a device for mixing a blood sample collected from a myelofibrosis patient, a reagent containing a solid phase on which WFA lectin is immobilized and a reagent containing a labeled anti-M2BP antibody to perform an antigen-antibody reaction, and a device for acquiring a signal based on the labeled anti-M2BP antibody specifically bound to WFA⁺-M2BP as a measured value of WFA⁺-M2BP, and
the computer comprising a memory for storing the measured value of WFA⁺-M2BP acquired by the immunoassay device and a predetermined cut-off value, and a processor for comparing the stored measured value of WFA⁺-M2BP with the stored predetermined cut-off value to determine the condition of myelofibrosis based on the comparison result.

Also disclosed herein is the use of the above immunoassay device and computer for manufacturing an apparatus for predicting the prognosis of myelofibrosis. Thus, the present disclosure provides:
the use of an immunoassay device and a computer for manufacturing an apparatus for determining the prognosis of myelofibrosis,
the immunoassay device comprising a device for mixing a blood sample collected from a myelofibrosis patient, a reagent containing a solid phase on which WFA lectin is immobilized and a reagent containing a labeled anti-M2BP antibody to perform an antigen-antibody reaction, and a device for acquiring a signal based on the labeled anti-M2BP antibody specifically bound to WFA⁺-M2BP as a measured value of WFA⁺-M2BP, and
the computer comprising a memory for storing the measured value of WFA⁺-M2BP acquired by the immunoassay device and a predetermined cut-off value, and a processor for comparing the stored measured value of WFA⁺-M2BP with the stored predetermined cut-off value to determine the prognosis of the patient based on the comparison result.

Further disclosed herein is the use of the above immunoassay device and computer for manufacturing an apparatus for monitoring the therapeutic effect on myelofibrosis. Thus, disclosed herein is:
the use of an immunoassay device and a computer for manufacturing an apparatus for monitoring the therapeutic effect of myelofibrosis,
the immunoassay device comprising a device for mixing a blood sample collected from a patient who has been treated for myelofibrosis, a reagent containing a solid phase on which WFA lectin is immobilized and a reagent containing a labeled anti-M2BP antibody to perform an antigen-antibody reaction, and a device for acquiring a signal based on the labeled anti-M2BP antibody specifically bound to WFA⁺-M2BP as a measured value of WFA⁺-M2BP, and
the computer comprising a memory for storing the measured value of WFA⁺-M2BP acquired by the immunoassay device and a predetermined cut-off value, and a processor for comparing the stored measured value of WFA⁺-M2BP with the stored predetermined cut-off value to determine the therapeutic effect on myelofibrosis based on the comparison result.

In the above-mentioned use, it is preferred that the immunoassay device comprises a device for mixing a blood sample, a reagent containing magnetic particles on which WFA lectin is immobilized and a reagent containing an enzyme-labeled anti-M2BP antibody to perform an antigen-antibody reaction, and a device for acquiring a chemiluminescent signal based on an enzyme-labeled antibody specifically bound to WFA⁺-M2BP as a measured value of WFA⁺-M2BP.

In the use described above, the computer may further comprise an input device for the user to input various commands, for example, a mouse, a keyboard, and the like. In addition, in the use described above, the computer may further comprise a display device for displaying the determination result, for example, a display device including an LCD, a CRT, and the like.

Hereinbelow, the present invention will be described in detail by examples, but the present invention is not limited to these examples.

### EXAMPLES

### Example 1: Determination of Condition of Myelofibrosis

### 1. Reagent

### (1-1) Buffer solution for sample dilution (first reagent)

A buffer solution containing 10 mM HEPES (pH 7.5) was prepared and used as a first reagent.

### (1-2) Magnetic particles on which WFA lectin is immobilized (second reagent)

A suspension containing magnetic particles on which WFA lectin was immobilized was prepared as follows and used as a second reagent.

### (1-2-1) Preparation of biotinylated dimeric WFA lectin

A WFA-containing solution (WFA concentration 2.5 mg/ml) was obtained by adding WFA lectin (manufactured by Vector Laboratories, trade name: Wisteria floribunda Lectin) to a 20 mM phosphate buffer solution (pH 7.5). 5-(N-Succinimidyloxycarbonyl)pentyl D-biotinamide (manufactured by DOJINDO LABORATORIES, trade name: Biotin-AC5-Osu) which is a crosslinking agent containing biotin was added to the obtained WFA-containing solution so that the WFA/crosslinking agent (molar ratio) would be 1/100. The resulting solution was incubated at 25°C for 90 minutes to react the WFA lectin with the crosslinking agent containing biotin to obtain a reaction product. The obtained reaction product was purified by high performance liquid chromatography (HPLC) to obtain a biotinylated dimeric WFA lectin. For HPLC, a phosphate buffer solution (pH 6.5) was used as an elution solvent, and a gel filtration column (manufactured by Tosoh Corporation, trade name: TSKgel G3000SWXL) was used as a separation column. Since the theoretical value of the molecular weight of the biotinylated tetrameric WFA lectin was 116,000, the predicted molecular weight of the obtained reaction product by HPLC was 56000, thus it is found that the reaction product is biotinylated dimeric WFA lectin.

### (1-2-2) Preparation of streptavidin-bound particle-containing liquid

Magnetic particles on which streptavidin was immobilized on its surface (average particle size 2 µm; the amount of streptavidin per 1 g of the magnetic particles is 2.9 to 3.5 mg. hereinafter also referred to as "STA-bound magnetic particles") were washed 3 times with a 10 mM HEPES buffer solution (pH 7.5). The washed STA-bound magnetic particles were added to a 10 mM HEPES buffer solution (pH 7.5) so as to have a streptavidin concentration of 18 to 22 µg/ml (a concentration of STA-bound magnetic particles of 0.48 to 0.52 mg/ml) to obtain STA-bonded magnetic particle-containing liquid.

### (1-2-3) Preparation of magnetic particles on which dimeric WFA lectin is immobilized

The biotinylated dimeric WFA lectin and the STA-bound magnetic particle-containing liquid were mixed so as to have a concentration of the biotinylated dimeric WFA lectin of 20 µg/ml and reacted. The resulting product was washed three times with a 100 mM MES buffer solution (pH 6.5) to obtain magnetic particles on which the dimeric WFA lectin was immobilized (hereinafter also referred to as "dimeric WFA-immobilized particles"). The obtained dimeric WFA-immobilized carrier was suspended in a 10 mM HEPES buffer solution (pH 7.5) to obtain a suspension containing dimeric WFA-immobilized particles (particle concentration: 0.5 w/v%).

### (1-3) Solution containing labeled anti-M2BP antibody (third reagent)

An alkaline phosphatase-labeled anti-M2BP monoclonal antibody (hereinafter also referred to as "ALP-labeled anti-M2BP antibody") was used as the labeled anti-M2BP antibody. A solution containing ALP-labeled anti-M2BP antibody and 10 mM HEPES (pH 7.5) (antibody concentration: 0.1 U/ml) was prepared and used as a third reagent.

### (1-4) Buffer solution for measurement (fourth reagent)

HISCL R4 reagent (manufactured by Sysmex Corporation) was used as a fourth reagent.

### (1-5) Substrate (fifth reagent)

HISCL R5 reagent (manufactured by Sysmex Corporation) using CDP-Star (registered trademark) (Applied Biosystems) as an alkaline phosphatase chemiluminescent substrate was used as a fifth reagent.

### 2. Determination of Condition of Myelofibrosis

### (2-1) Target patient

Relationship between myelofibrosis and the WFA⁺-M2BP value in patients with polycythemia vera (PV), essential thrombocythemia (ET) and primary myelofibrosis (PMF) diagnosed at Nagoya City University Hospital was retrospectively analyzed. HBs antigen positive or HCV antibody positive cases were excluded. The condition of myelofibrosis of each patient was determined independently of the information on the measured value of WFA⁺-M2BP. Specifically, according to "European consensus on grading of bone marrow fibrosis", bone marrow biopsy specimens were evaluated by a hematologist and classified into MF-0 to MF-3. This study was subjected to IRB approval review at this hospital.

### (2-2) Measurement of M2BP having sugar chain that binds to WFA lectin

As the blood sample, the stored serum of the above patient was used. WFA⁺-M2BP was measured with a fully automated immunoassay system HISCL2000i (manufactured by Sysmex Corporation) using the first to fifth reagents described above. In Example 1, as the measured value of WFA⁺-M2BP, the values calculated from signal intensities of the blood sample of the patient, HISCL M2BPGi NC (manufactured by Sysmex Corporation, hereinafter referred to as "NC sample") and HISCL M2BPGi PC (manufactured by Sysmex Corporation, hereinafter referred to as "PC sample") were used. This measured value is automatically calculated by HISCL2000i according to the following calculation formula.

WFA⁺-M2BP measured value = [(Signal intensity of blood sample of patient) - (Signal intensity of NC sample)]/[(Signal intensity of PC sample) - (Signal intensity of NC sample)]

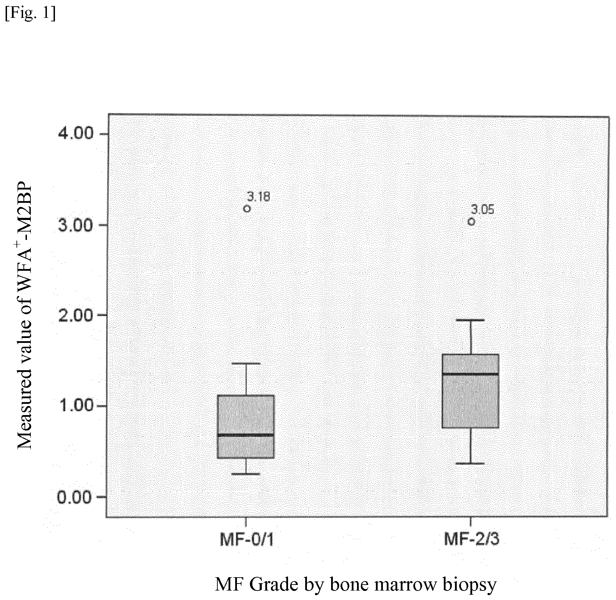

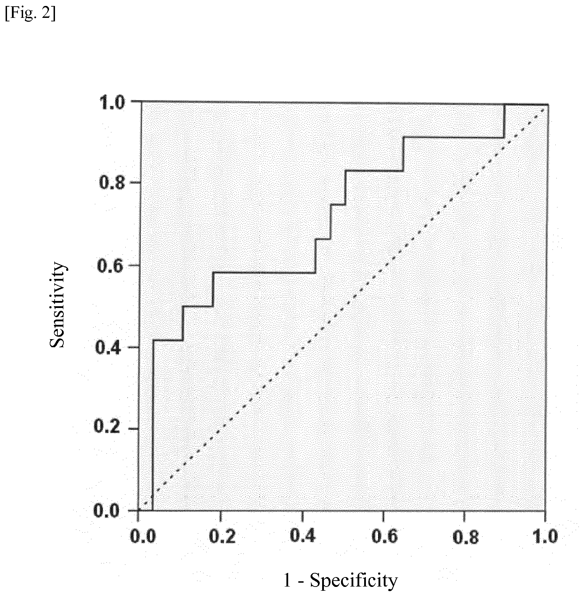

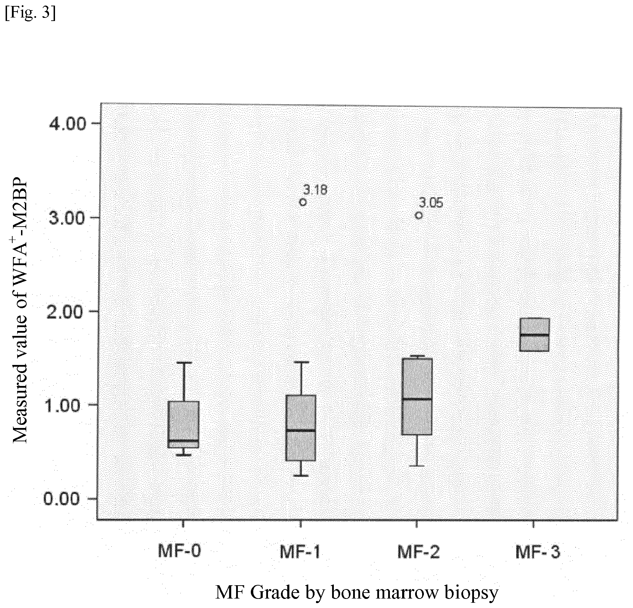

The measured values of WFA⁺-M2BP were compared between the MF-0/1 group and the MF-2/3 group classified by bone marrow biopsy, using Mann-Whitney U test. The cut-off value of WFA⁺-M2BP was determined from the ROC curve, and the sensitivity, specificity, positive predictive value and negative predictive value were calculated. SPSS statistical software (manufactured by IBM) was used for data analysis, and it was determined as statistically significant when the p value was less than 0.05. The measured values of WFA⁺-M2BP in each of the MF-0/1 group and the MF-2/3 group are shown in Fig. 1, and the measured values of WFA⁺-M2BP in each group of MF-0 to MF-3 are shown in Fig. 3. Also, the ROC curve is shown in Fig. 2.

### 3. Results

Patient background of total of 40 examples is shown in Tables 1 and 2. Table 1 is a table in which patients are classified into two groups of MF-0/1 and MF-2/3, and Table 2 is a table in which patients are classified into four groups of MF-0 to MF-3. The measured values of WFA⁺-M2BP listed in Tables 1 and 2 are median values of each group. The breakdown of the diseases is 10 cases (25%) of PV, 27 cases (68%) of ET, 3 cases (8%) of PMF, and the breakdown of the secondary myelofibrosis is 1 case of post-PV MF, and 4 cases of post-ET MF. There were 20 men and 20 females. The median age was 72 years (range: 28 to 89 years). JAK2V617F mutation was detected in 26 cases (65%). There were 19 cases (48%) showing splenomegaly detected by CT. There were 14 cases (including treatment with antiplatelet medicine) with no treatment and only follow-up, 23 cases with hydroxyurea (HU) oral administration, and 3 cases using a JAK inhibitor. In the evaluation by bone marrow biopsy, there were 3 cases (8%) of MF-0, 25 cases (63%) of MF-1, 10 cases (25%) of MF-2, and 2 cases (5%) of MF-3. The median of the measured values of total of 40 examples of WFA⁺-M2BP was 0.86 (range: 0.25 to 3.18).

**[Table 1]**

| | MF-0/1 | MF-2/3 | |
|---|---|---|---|
| | n=28 | n=12 | p-Value |
| MF Grade | | | - |
| MF-0 | 3 | | |
| MF-1 | 25 | | |
| MF-2 | | 10 | |
| MF-3 | | 2 | |
| Median of age (range) | 71 (28-89) | 74 (49-85) | 0.983 |
| Gender (male/female) | 13/15 | 7/5 | 0.731 |
| Underlying disease | | | 0.042 |
| PV | 8 | 2 | |
| ET | 20 | 7 | |
| PMF | 0 | 3 | |
| Secondary myelofibrosis | 0 | 5 | 0.001 |
| JAK2 mutation (+) | 20 | 6 | 0.281 |
| Splenomegaly | 9 | 10 | 0.005 |
| Leukocyte (/µL) | 6,850 | 9,550 | 0.545 |
| (Range) | (2,200-17,600) | (2,500-28,200) | |
| Appearance of blast | 0 | 3 | 0.022 |
| Appearance of erythroblast | 0 | 3 | 0.022 |
| Hemoglobin (g/dL) (Range) | 13.4 (8.4-22.0) | 9.7 (6.8-15.4) | 0.001 |
| Platelet (×10⁴/µL) (Range) | 64.7 (20.4-133.0) | 30.4 (4.1-124.4) | 0.114 |
| LDH (U/L) (Range) | 248 (163-520) | 406 (177-934) | 0.026 |
| Measured value of WFA⁺-M2BP | 0.68 | 1.36 | 0.030 |
| (Range) | (0.25-3.18) | (0.36-3.05) | |
| Treatment | | | - |
| No treatment | 9 | 5 | |
| HU | 19 | 4 | |
| JAK Inhibitor | 0 | 3 | |

**[Table 2]**

| | MF-0 | MF-1 | MF-2 | MF-3 |
|---|---|---|---|---|
| | n=3 | n=25 | n=10 | n=2 |
| Median of age (range) | 82 (65-85) | 71 (28-89) | 75 (49-85) | 73 (72-74) |
| Gender (male/female) | 2/1 | 11/14 | 5/5 | 2/0 |
| Underlying disease | | | | |
| PV | 1 | 7 | 1 | 1 |
| ET | 2 | 18 | 6 | 1 |
| PMF | 0 | 0 | 3 | 0 |
| Secondary myelofibrosis | 0 | 0 | 3 | 2 |
| JAK2 mutation (+) | 1 | 19 | 6 | 0 |
| Splenomegaly | 1 | 8 | 8 | 2 |
| Leukocyte (/µL) | 5.100 | 6,900 | 10.750 | 3.050 |
| (Range) | (4,900-17,300) | (2,200-17,600) | (3,200-28,200) | (2,500-3,600) |
| Appearance of blast | 0 | 0 | 2 | 1 |
| Appearance of etythroblast | 0 | 0 | 1 | 2 |
| Hemoglobin (g/dL) (Range) | 12.1 (10.4-17.4) | 13.5 (8.4-22.0) | 10.6 (7.3-15.4) | 7.1 (6.8-7.4) |
| Platelet (×10⁴/µL) (Range) | 46.9 (35.5-78.4) | 65.2 (20.4-133.0) | 30.4 (4.1-124.4) | 37.9 (19.2-56.5) |
| LDH (U/L) (Range) | 246 (211-254) | 248 (163-520) | 406 (177-663) | 598 (262-934) |
| Measured value of WFA⁺-M2BP | 0.62 | 0.73 | 1.08 | 1.78 |
| (Range) | (0.47-1.46) | (0.25-3.18) | (0.36-3.05) | (1.60-1.95) |
| Treatment | | | | |
| No treatment | 1 | 8 | 5 | 0 |
| HU | 2 | 17 | 3 | 1 |
| JAK Inhibitor | 0 | 0 | 2 | 1 |

Comparing the patient background between the MF-0/1 group and the MF-2/3 group, the MF-2/3 group often showed more increases in PMF, secondary MF, splenomegaly, appearance of blasts and erythroblasts in the peripheral blood, anemia, and lactate dehydrogenase (LDH) (see Table 1). In addition, comparing the measured values of WFA⁺-M2BP, it was significantly higher in the MF-2/3 group than in the MF-0/1 group (p = 0.030). The cut-off value of the measured value of WFA⁺-M2BP obtained from the ROC curve was 1.24 (see Fig. 2). When whether the condition of myelofibrosis of each patient corresponds to MF-2/3 was determined using the cut-off value of the measured value of WFA⁺-M2BP, the sensitivity was 58.3%, the specificity was 82.1%, the positive predictive value was 58.3%, and the negative predictive value was 82.1%. Therefore, the probability that WFA⁺-M2BP contained in the peripheral blood of an MF patient becomes a marker for determining the condition of myelofibrosis was indicated.

### Example 2: Monitoring of Therapeutic Effect on Myelofibrosis

### 1. Materials

### (1-1) Reagents and measurement device

As reagents for measuring WFA⁺-M2BP in serum, the first to fifth reagents as in Example 1 were used. A fully automated immunoassay system HISCL2000i (manufactured by Sysmex Corporation) was used as a measurement device.

### (1-2) Therapeutic agent

For the treatment of MF, ruxolitinib (manufactured by Novartis Pharma Stein AG, preparation name: Jakafi (registered trademark) tablet) which is a therapeutic agent for myelofibrosis (JAK inhibitor) was used. The dose was determined according to the condition of the patient, according to the package insert of the preparation.

### 2. Monitoring of therapeutic effect

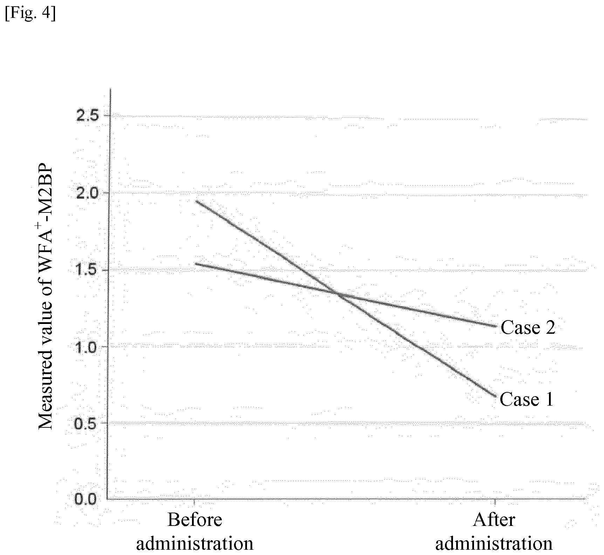

Two cases of patients with myelofibrosis (72-year-old male and 66-year-old female) were targeted. The chief complaint of the patients was abdominal distension accompanied by prominent splenomegaly. Peripheral blood was collected from each patient before administration of a JAK inhibitor, and WFA⁺-M2BP in the serum was measured in the same manner as in Example 1. A therapeutic agent was administered to the patients for 4 or 6 months. As a result, the patient's spleen contracted, and accompanying symptoms improved. At this point, peripheral blood was collected from each patient, and WFA⁺-M2BP in the serum was measured. The results are shown in Fig. 4. In the figure, Case 1 shows a patient to whom the therapeutic agent was administered for 6 months, and Case 2 shows a patient to whom the therapeutic agent was administered for 4 months.

### 3. Results

As shown in Fig. 4, in Cases 1 and 2, it was confirmed that the measured value of serum WFA⁺-M2BP after administration of the therapeutic agent decreased as compared with that before administration. Especially for Case 1, it was confirmed that the condition of myelofibrosis was MF-2/3 and the WFA⁺-M2BP measured value was as high as 1.96 before administration of the therapeutic agent, but after administration of the therapeutic agent, the WFA⁺-M2BP measured value was improved to 0.68 which corresponds to MF-0/1. In Cases 1 and 2, accompanying symptoms such as splenomegaly have improved. Because it is difficult to perform highly invasive bone marrow biopsy in a short time, improvement of fibrosis is not histopathologically confirmed, and it is studies of a small number of cases, thus the possibility of selective bias cannot be denied. However, it has been considered that the clinical course of these two cases suggests the possibility of determining the effect of treatment on myelofibrosis, based on the measured value of WFA⁺-M2BP.

### Example 3: Monitoring of Therapeutic Effect on Myelofibrosis (2)

### 1. Materials

### (1-1) Reagents and measurement device

As reagents for measuring WFA⁺-M2BP in serum, the first to fifth reagents as in Example 1 were used. A fully automated immunoassay system HISCL2000i (manufactured by Sysmex Corporation) was used as a measurement device.

### (1-2) Therapeutic agent

As in Example 2, for the treatment of MF, ruxolitinib (manufactured by Novartis Pharma Stein AG, preparation name: Jakafi (registered trademark) tablet) which is a therapeutic agent for myelofibrosis (JAK inhibitor) was used. The dose was determined according to the condition of the patient, according to the package insert of the preparation.

### 2. Monitoring of therapeutic effect

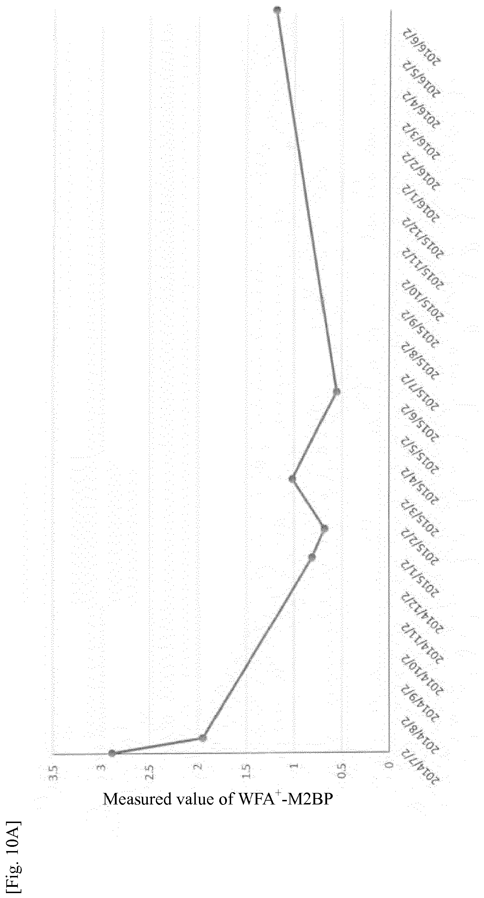

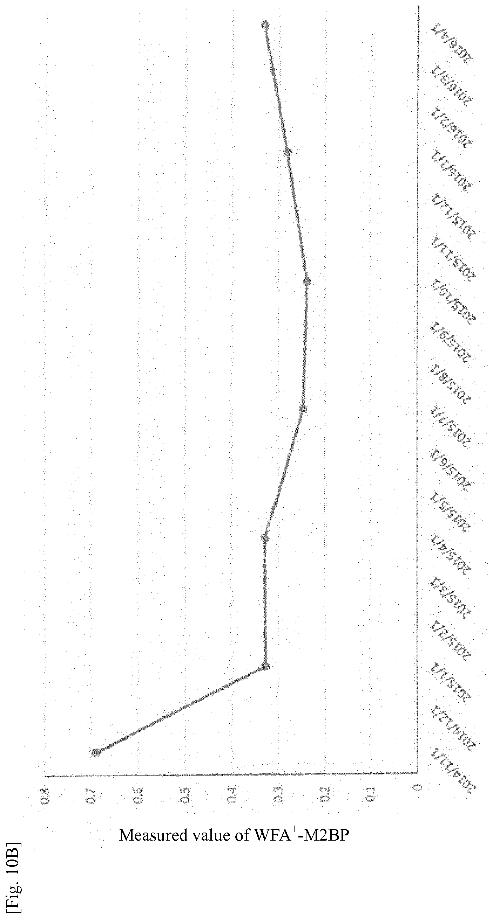

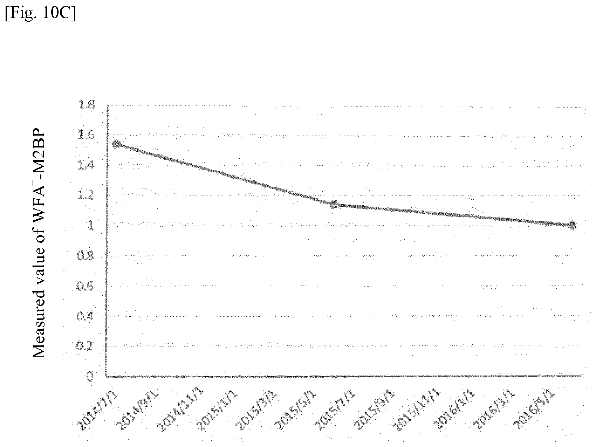

Three cases of myelofibrosis patients (patient A: 74-year-old male, patient B: 81-year-old male, and patient C: 68-year-old female) who were patients different from the patients in Example 2 were targeted. The chief complaint of the patients was abdominal distension accompanied by prominent splenomegaly. Peripheral blood was collected from each patient before administration of a therapeutic agent, and WFA⁺-M2BP in the serum was measured in the same manner as in Example 1. Administration of a therapeutic agent to Patient A was started on July 23, 2014, peripheral blood was sampled periodically until June 2, 2016, and WFA⁺-M2BP in the serum was measured. Administration of a therapeutic agent to Patient B was started on November 18, 2014, peripheral blood was collected periodically until April 1, 2016, and WFA⁺-M2BP in the serum was measured. Administration of a therapeutic agent to Patient C was started on February 16, 2015, peripheral blood was collected periodically until May 1, 2016, and WFA⁺-M2BP in the serum was measured. In addition, after administration of the therapeutic agent, whether or not the patient's splenomegaly improved was confirmed by findings. The measurement results of WFA⁺-M2BP are shown in Figs. 10A to 10C.

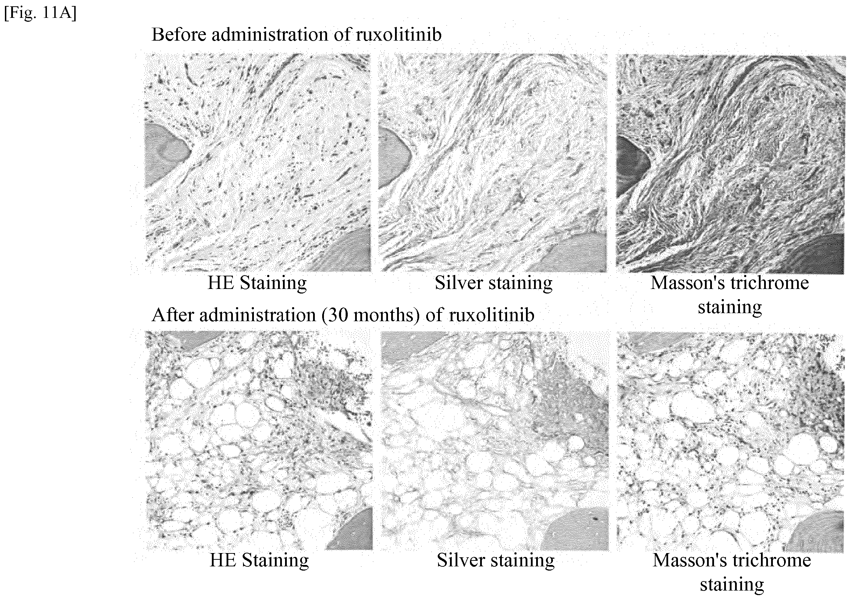

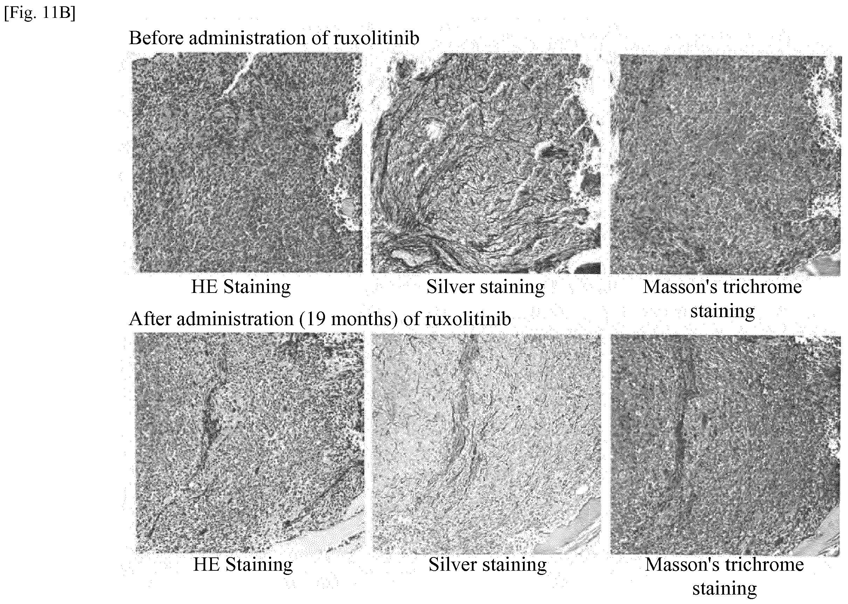

For patients A and C, bone marrow biopsy was performed before and after administration of the therapeutic agent, and whether or not fibrosis was improved was confirmed, specifically, as follows. From patient A, bone marrow was collected before administration and 30 months after administration of the therapeutic agent. Bone marrow was also collected from patient C before administration and after 19 months after administration of the therapeutic agent. Hematoxylin staining, silver staining and Masson's trichrome staining were performed on the collected bone marrow by an ordinary method. Obtained pathological tissue specimens were observed to evaluate fibrosis of bone marrow. Micrographs of the stained bone marrow are shown in Figs. 11A and 11B.

### 3. Results

As shown in Figs. 10A to 10C, it was confirmed that the measured value of serum WFA⁺-M2BP decreased as compared with that before administration due to continuous administration of the therapeutic agent in all patients. In addition, it was confirmed by findings that splenomegaly improved in all patients after administration of the therapeutic agent. That is, the decrease in the measured value of serum WFA⁺-M2BP by administration of the therapeutic agent was consistent with the findings of improvement in splenomegaly. As shown in Fig. 11A, the condition of myelofibrosis of patient A was MF-3 before administration of the therapeutic agent, but it improved to MF-2 equivalent after administration of the therapeutic agent. In addition, as shown in Fig. 11B, the condition of myelofibrosis of patient C was MF-2 before administration of the therapeutic agent, but it improved to MF-1 after administration of the therapeutic agent. Therefore, the decrease in the measured value of serum WFA⁺-M2BP by administration of the therapeutic agent was consistent with the result of bone marrow biopsy. From these facts, it was shown that the measured value of serum WFA⁺-M2BP is an objective indicator for monitoring the therapeutic effect on myelofibrosis by administration of the therapeutic agent.

### REFERENCE SIGNS LIST

- 10: Diagnostic apparatus
- 20: Immunoassay device
- 30: Computer system
- 40: Recording medium
- 300: Computer body
- 301: Input unit
- 302: Display unit
- 310: CPU
- 311: ROM
- 312: RAM
- 313: Hard disk
- 314: Input/output interface
- 315: Reading device
- 316: Communication interface
- 317: Image output interface
- 318: bus

## Claims

1. A method for assisting the diagnosis of the condition of myelofibrosis, comprising the steps of:
measuring a marker protein contained in the peripheral blood collected from a patient with myelofibrosis; and
determining the condition of myelofibrosis of the patient based on the measured value of the marker protein, wherein
the marker protein is M2BP (Mac-2-binding protein) having a sugar chain that binds to WFA (Wisteria floribunda agglutinin) lectin.

2. The method according to claim 1, wherein determining the condition of myelofibrosis comprises determining whether it is a grade corresponding to MF-0/1 or a grade corresponding to MF-2/3, among the grades of myelofibrosis represented by MF-0, MF-1, MF-2 and MF-3.

3. The method according to claim 1, which comprises determining to which grade the condition of myelofibrosis corresponds, among the grades of myelofibrosis represented by MF-0, MF-1, MF-2 and MF-3.

4. The method according to claim 2, wherein
when the measured value of the marker protein is equal to or higher than a predetermined cut-off value, the condition of myelofibrosis is determined to be a grade corresponding to MF-2/3, and
when the measured value of the marker protein is lower than the predetermined cut-off value, the condition of myelofibrosis is determined to be a grade corresponding to MF-0/1.

5. The method according to any one of claims 1 to 4, wherein the marker protein is measured using WFA lectin and an anti-M2BP antibody.

6. The method according to claim 5, wherein the WFA lectin is immobilized on a magnetic particle and the anti-M2BP antibody is labeled with a labeling substance.

7. The method according to any one of claims 1 to 6, wherein the marker protein is measured using a magnetic particle on which dimeric WFA lectin is immobilized, and an anti-M2BP antibody.

8. A method for monitoring the therapeutic effect on myelofibrosis, comprising the steps of:
measuring a marker protein contained in the peripheral blood collected from a patient with myelofibrosis who has been treated for myelofibrosis; and
determining the therapeutic effect on myelofibrosis based on the measured value of the marker protein, wherein
the marker protein is M2BP (Mac-2-binding protein) having a sugar chain that binds to WFA (Wisteria floribunda agglutinin) lectin.

9. The method according to claim 8, wherein the step of determining the therapeutic effect is to determine a therapeutic effect on myelofibrosis by comparing the measured value of the marker protein with the measured value of the marker protein of the patient measured in the past.

10. The method according to claim 8, wherein the step of monitoring the therapeutic effect is to determine a therapeutic effect on myelofibrosis by determining the condition of myelofibrosis of the patient based on the measured value of the marker protein.

11. The method according to claim 8 or 9, wherein the treatment is drug therapy or non-drug therapy.

12. The method according to claim 10 or 11, wherein the treatment is drug therapy using ruxolitinib.

13. The method according to claim 10 or 11, wherein the treatment is non-drug therapy selected from blood transfusion therapy, bone marrow transplantation, splenectomy and radiotherapy.

14. An apparatus for diagnosing the condition of myelofibrosis, comprising:
a computer containing a processor and a memory under the control of the processor, wherein
a computer program for making the computer execute the steps of:
acquiring a measured value of the marker protein contained in the peripheral blood collected from a patient with myelofibrosis; and
determining the condition of myelofibrosis of the patient based on the measured value of the marker protein that is recorded in the memory, and wherein
the marker protein is M2BP (Mac-2-binding protein) having a sugar chain that binds to WFA (Wisteria floribunda agglutinin) lectin.

15. An apparatus for monitoring the therapeutic effect on myelofibrosis, comprising: a computer containing a processor and a memory under the control of the processor, wherein
a computer program for making the computer execute the steps of:
acquiring a measured value of the marker protein contained in the peripheral blood collected from a patient with myelofibrosis who has been treated for myelofibrosis; and determining the therapeutic effect on myelofibrosis based on the measured value of the marker protein that is recorded in the memory, and wherein the marker protein is M2BP (Mac-2-binding protein) having a sugar chain that binds to WFA (Wisteria floribunda agglutinin) lectin.

## Patentansprüche

1. Verfahren zur Unterstützung der Diagnose des Zustands von Myelofibrose, umfassend die Schritte:
Messen eines Markerproteins, das in dem von einem Patienten mit Myelofibrose gesammeltem peripherem Blut enthalten ist;
und
Bestimmen des Zustands von Myelofibrose des Patienten auf Grundlage des Messwerts des Markerproteins, wobei das Markerprotein M2BP (Mac-2-bindendes Protein) mit einer Zuckerkette, die an WFA(Wisteria-floribunda-Agglutinin)-Lectin bindet, ist.

2. Verfahren gemäß Anspruch 1, wobei Bestimmen des Zustands von Myelofibrose umfasst, zu bestimmen, ob sie von einem Grad, der MF-0/1 entspricht, oder einem Grad, der MF-2/3 entspricht, unter den durch MF-0, MF-1, MF-2 und MF-3 dargestellten Graden von Myelofibrose ist.

3. Verfahren gemäß Anspruch 1, umfassend Bestimmen, welchem Grad der Zustand von Myelofibrose unter den durch MF-0, MF-1, MF-2 und MF-3 dargestellten Graden von Myelofibrose entspricht.

4. Verfahren gemäß Anspruch 2, wobei,
wenn der Messwert des Markerproteins gleich einem oder größer als ein vorbestimmter Grenzwert ist, der Zustand von Myelofibrose als ein Grad, der MF-2/3 entspricht, bestimmt wird, und
wenn der Messwert des Markerproteins kleiner als der vorbestimmte Grenzwert ist, der Zustand von Myelofibrose als ein Grad, der MF-0/1 entspricht, bestimmt wird.

5. Verfahren gemäß einem der Ansprüche 1 bis 4, wobei das Markerprotein unter Verwendung von WFA-Lectin und eines Anti-M2BP-Antikörpers gemessen wird.

6. Verfahren gemäß Anspruch 5, wobei das WFA-Lectin auf einem magnetischen Partikel immobilisiert ist und der Anti-M2BP-Antikörper mit einem Markierungsstoff markiert ist.

7. Verfahren gemäß einem der Ansprüche 1 bis 6, wobei das Markerprotein unter Verwendung eines magnetischen Partikels, auf dem dimeres WFA-Lectin immobilisiert ist, und eines Anti-M2BP-Antikörpers gemessen wird.

8. Verfahren zur Überwachung der therapeutischen Wirkung auf Myelofibrose, umfassend die Schritte:
Messen eines Markerproteins, das in dem von einem Patienten mit Myelofibrose, der auf Myelofibrose behandelt worden ist, gesammeltem peripherem Blut enthalten ist; und
Bestimmen der therapeutischen Wirkung auf Myelofibrose auf Grundlage des Messwerts des Markerproteins, wobei das Markerprotein M2BP (Mac-2-bindendes Protein) mit einer Zuckerkette, die an WFA(Wisteria-floribunda-Agglutinin)-Lectin bindet, ist.

9. Verfahren gemäß Anspruch 8, wobei der Schritt des Bestimmens der therapeutischen Wirkung ist, eine therapeutische Wirkung auf Myelofibrose durch Vergleichen des Messwerts des Markerproteins mit dem in der Vergangenheit gemessenen Messwert des Markerproteins des Patienten zu bestimmen.

10. Verfahren gemäß Anspruch 8, wobei der Schritt des Überwachens der therapeutischen Wirkung ist, eine therapeutische Wirkung auf Myelofibrose durch Bestimmen des Zustands von Myelofibrose des Patienten auf Grundlage des Messwerts des Markerproteins zu bestimmen.

11. Verfahren gemäß Anspruch 8 oder 9, wobei die Behandlung Arzneimitteltherapie oder Nichtarzneimittel-Therapie ist.

12. Verfahren gemäß Anspruch 10 oder 11, wobei die Behandlung Arzneimitteltherapie unter Verwendung von Ruxolitinib ist.

13. Verfahren gemäß Anspruch 10 oder 11, wobei die Behandlung Nichtarzneimittel-Therapie ausgewählt aus Bluttransfusionstherapie, Knochenmarktransplantation, Splenektomie und Strahlentherapie ist.

14. Vorrichtung zur Diagnose des Zustands von Myelofibrose, umfassend:
einen Computer, der einen Prozessor und einen Speicher unter der Steuerung des Prozessors umfasst, wobei
ein Computerprogramm zum Bewirken, dass der Computer die Schritte ausführt:
Aufnehmen eines Messwerts des Markerproteins, das in dem von einem Patienten mit Myelofibrose gesammeltem peripherem Blut enthalten ist; und
Bestimmen des Zustands von Myelofibrose des Patienten auf Grundlage des Messwerts des Markerproteins,
in dem Speicher gespeichert ist, und wobei
das Markerprotein M2BP (Mac-2-bindendes Protein) mit einer Zuckerkette, die an WFA(Wisteria-floribunda-Agglutinin)-Lectin bindet, ist.

15. Vorrichtung zum Überwachen der therapeutischen Wirkung auf Myelofibrose, umfassend: einen Computer, der einen Prozessor und einen Speicher unter der Steuerung des Prozessors umfasst, wobei
ein Computerprogramm zum Bewirken, dass der Computer die Schritte ausführt:
Aufnehmen eines Messwerts des Markerproteins, das in dem von einem Patienten mit Myelofibrose, der auf Myelofibrose behandelt worden ist, gesammeltem peripherem Blut enthalten ist; und
Bestimmen der therapeutischen Wirkung auf Myelofibrose auf Grundlage des Messwerts des Markerproteins,
in dem Speicher gespeichert ist, und wobei
das Markerprotein M2BP (Mac-2-bindendes Protein) mit einer Zuckerkette, die an WFA(Wisteria-floribunda-Agglutinin)-Lectin bindet, ist.

## Revendications

1. Procédé destiné à assister le diagnostic de la condition de myélofibrose, comprenant les étapes de :
mesure d'une protéine marqueur contenue dans le sang périphérique prélevé chez un patient atteint de myélofibrose ; et
détermination de la condition de myélofibrose du patient sur la base de la valeur mesurée de la protéine marqueur,
dans lequel
la protéine marqueur est la M2BP (protéine de liaison à Mac-2) ayant une chaîne glucidique qui se lie à une lectine WFA (agglutinine de Wisteria floribunda).

2. Procédé selon la revendication 1, dans lequel la détermination de la condition de myélofibrose comprend une détermination de s'il s'agît d'un grade correspondant à MF-0/1 ou d'un grade correspondant à MF-2/3, parmi les grades de myélofibrose représentés par MF-0, MF-1, MF-2 et MF-3.

3. Procédé selon la revendication 1, qui comprend la détermination du grade auquel correspond la condition de myélofibrose, parmi les grades de myélofibrose représentés par MF-0, MF-1, MF-2 et MF-3.

4. Procédé selon la revendication 2, dans lequel lorsque la valeur mesurée de la protéine marqueur est supérieure ou égale à une valeur seuil prédéterminée, la condition de myélofibrose est déterminée comme étant à un grade correspondant à MF-2/3, et
lorsque la valeur mesurée de la protéine marqueur est inférieure à la valeur seuil prédéterminée, la condition de myélofibrose est déterminée comme étant à un grade correspondant à MF-0/1.

5. Procédé selon l'une quelconque des revendications 1 à 4, dans lequel la protéine marqueur est mesurée en utilisant une lectine WFA et un anticorps anti-M2BP.

6. Procédé selon la revendication 5, dans lequel la lectine WFA est immobilisée sur une particule magnétique et l'anticorps anti-M2BP est marqué avec une substance marqueur.

7. Procédé selon l'une quelconque des revendications 1 à 6, dans lequel la protéine marqueur est mesurée en utilisant une particule magnétique, sur laquelle une lectine WFA dimérique est immobilisée, et un anticorps anti-M2BP.

8. Procédé destiné à suivre l'effet thérapeutique sur la myélofibrose, comprenant les étapes de :
mesure d'une protéine marqueur contenue dans le sang périphérique prélevé chez un patient atteint de myélofibrose qui a été traité pour une myélofibrose ; et
détermination de l'effet thérapeutique sur la myélofibrose, sur la base de la valeur mesurée de la protéine marqueur, dans lequel
la protéine marqueur est M2BP (protéine de liaison à Mac-2) ayant une chaîne glucidique qui se lie à une lectine WFA (agglutinine de Wisteria floribunda).

9. Procédé selon la revendication 8, dans lequel l'étape de détermination de l'effet thérapeutique est pour déterminer un effet thérapeutique sur la myélofibrose par comparaison de la valeur mesurée de la protéine marqueur avec la valeur mesurée de la protéine marqueur du patient mesurée par le passé.

10. Procédé selon la revendication 8, dans lequel l'étape de suivre l'effet thérapeutique est pour déterminer un effet thérapeutique sur la myélofibrose par détermination la condition de myélofibrose du patient sur la base de la valeur mesurée de la protéine marqueur.

11. Procédé selon les revendications 8 ou 9, dans lequel le traitement est une thérapie médicamenteuse ou une thérapie non médicamenteuse.

12. Procédé selon les revendications 10 ou 11, dans lequel le traitement est une thérapie médicamenteuse utilisant le ruxolitinib.

13. Procédé selon les revendications 10 ou 11, dans lequel le traitement est une thérapie non médicamenteuse choisie parmi une thérapie par transfusion sanguine, greffe de moelle osseuse, splénectomie et radiothérapie.

14. Appareil pour diagnostiquer la condition de myélofibrose, comprenant :
un ordinateur contenant un processeur et une mémoire sous le contrôle du processeur, dans lequel
un programme informatique permettant à l'ordinateur exécute les étapes de:
acquérir une valeur mesurée de la protéine marqueur contenue dans le sang périphérique prélevé chez un patient atteint de myélofibrose ; et
déterminer la condition de myélofibrose du patient, sur la base de la valeur mesurée de la protéine marqueur qui est enregistrée dans la mémoire, et dans lequel la protéine marqueur est M2BP (protéine de liaison à Mac-2) ayant une chaîne glucidique qui se lie à une lectine WFA (agglutinine de Wisteria floribunda).

15. Appareil pour surveiller l'effet thérapeutique sur une myélofibrose, comprenant : un ordinateur contenant un processeur et une mémoire sous le contrôle du processeur, dans lequel
un programme informatique permettant à l'ordinateur d'exécuter les étapes de:
acquérir une valeur mesurée de la protéine marqueur contenue dans le sang périphérique prélevé chez un patient atteint de myélofibrose qui a été traité pour la myélofibrose ; et
déterminer l'effet thérapeutique sur la myélofibrose, sur la base de la valeur mesurée de la protéine marqueur qui est enregistrée dans la mémoire, et dans laquelle la protéine marqueur est M2BP (protéine de liaison à Mac-2) ayant une chaîne glucidique qui se lie à une lectine WFA (agglutinine de Wisteria floribunda).
